# EUROPEAN PATENT SPECIFICATION

(11) **EP 2 853 281 B1**
(45) Date of publication and mention of the grant of the patent: **23.11.2016**
(21) Application number: 14173723.9
(22) Date of filing: 24.06.2014
(51) Int. Cl.: A61M 25/00, A61M 25/09

(54) **Guide wire**
Führungsdraht
Fil-guide

(30) Priority: 25.09.2013 JP 2013197686
(43) Date of publication of application: 01.04.2015
(73) Proprietor: ASAHI INTECC CO., LTD., Nagoya-shi, Aichi 463-0024 (JP)
(72) Inventor: Takada, Keigo, Nagoya-shi, Aichi 463-0024 (JP)
(74) Representative: Winter, Brandl, Fürniss, Hübner, Röss, Kaiser, Polte - Partnerschaft mbB

(56) References cited:
- EP-A1- 0 982 046
- EP-A1- 1 875 941
- EP-A2- 2 371 402
- US-A1- 2006 047 224

## Description

### Technical Field

The present invention relates to a guide wire that is inserted into a lumen such as a blood vessel.

### Background Art

Known is a guide wire used when inserting a catheter into a blood vessel. When inserting a catheter, first the guide wire is inserted into the blood vessel, and then the catheter is allowed to proceed along the guide wire. In such manner, the guide wire functions as a guide which guides the catheter to the lesion area.

As such guide wire, commonly used is a so-called coil-type guide wire having the distal end portion of its core shaft covered with a coil body. In addition, for the purpose of increasing lubricity within the blood vessel, proposed is a guide wire having the surface of its coil body covered with a hydrophilic coating film (cf. US 5,840,046 or JP 2008-237621 A). The hydrophilic coating film is capable of exhibiting sufficient lubricity in cases when moisture (such as blood) exists in its surroundings.

Furthermore, US 2006/0047224 A1, EP 1 875 ,941 A1, EP 0 982 046 A1, and EP 2 371 402 A2 disclose guide wires having the surface of a coil body covered with a multilayer coating of various hydrophobic and hydrophilic materials.

However, with the abovementioned conventional guide wire, in environments where it is difficult for blood to be supplied (environments where there is little moisture surrounding the guide wire), for example inside the lesion of a chronic total occlusion (CTO), there was the problem of sufficient lubricity not being exhibited due to stagnation of the supply of moisture to the hydrophilic coating film.

### Summary of Invention

### Technical Problem

The present invention has been devised in light of the abovementioned problem exhibited by conventional technology and the object of the present invention is to provide technology for a guide wire having its coil body covered with a hydrophilic coating film capable of exhibiting sufficient lubricity even in a situation where there is little moisture in its surroundings.

### Solution to Problem

In order to solve the abovementioned problem, a guide wire according to the present invention, as described in the claims, uses the following configuration. Namely, a guide wire comprising a core shaft, a coil body which covers said core shaft, and a coating portion which covers said coil body, wherein said coating portion is formed by stacking two layer films, wherein the guide wire is characterized in that the lowermost layer film of said coating portion is a hydrophobic film, the upper layer film above the lowermost layer film of said coating portion is a hydrophilic film, said hydrophobic film is arranged to come between the wires of said coil body, and a space is formed between the hydrophilic film and the sections of the hydrophobic film where said hydrophobic film is arranged to come between the wires of said coil body.

Here, the "hydrophilic film" of the present invention is not limited to a film formed of a hydrophilic substance (such as a hydrophilic resin) and includes any film that is formed of a hydrophilic substance (such as a hydrophilic resin) and a hydrophobic substance (such as a cross-linking agent) and exhibits hydrophilic properties as a whole depending on the mixing ratio of the substances. In addition, the "hydrophobic film" of the present invention is not limited to a film formed of a hydrophobic substance (such as a hydrophobic resin) and includes any film that is formed of a hydrophilic substance (such as a hydrophilic resin) and a hydrophobic substance (such as a cross-linking agent) and exhibits hydrophobic properties as a whole depending on the mixing ratio of the substances.

With such guide wire of the present invention, as spaces are formed between the hydrophilic film and the sections in which the hydrophobic film are arranged to come between the wires of the coil body, it is possible to pool moisture within these spaces. Accordingly, even in an environment where there is little moisture surrounding the guide wire such as inside the lesion of a chronic total occlusion (CTO) and supply of moisture to the hydrophilic film is stagnated, it is possible to supply the moisture pooled in the space to the hydrophilic film. As a result, it becomes possible to sufficiently exhibit lubricity of the hydrophilic film of the guide wire.

In addition, in the abovementioned guide wire according to the present invention, the hydrophilic film of the coating portion may be that of 2 or more layers while the hydrophilic film of the top layer has a higher degree of water retentivity than the hydrophilic film of the bottom layer.

Even with such guide wire according to the present invention, in an environment where there is little moisture in the surroundings, by supplying the moisture pooled in the spaces to the hydrophilic film, it is possible to sufficiently exhibit the lubricity thereof.

In addition, as the hydrophilic film of the coating portion is that of 2 or more layers while the hydrophilic film of the top layer has a higher degree of water retentivity than the hydrophilic film of the bottom layer, it is possible to ensure that moisture is pooled in the spaces. Accordingly, for example, in a case where the guide wire (and coil body) is largely bent inside a blood vessel, it is possible to suppress the moisture pooled in the spaces from being discharged to the outside. As a result, in a case where moisture surrounding the guide wire becomes less, it becomes possible to ensure that moisture is supplied to the hydrophilic film.

In addition, with the guide wire according to the present invention, in cases when the hydrophilic film of the coating portion is that having 2 or more layers, in the sections where the films of the coating portion are arranged to come between the wires of the coil body, spaces may also be formed between the hydrophilic films.

With such guide wire according to the present invention, not only are spaces formed between the hydrophobic film (film of the lowermost layer) and the hydrophilic film but spaces are also formed between the hydrophilic films of the top layers thereof. Accordingly, as there are a greater number of spaces, it is possible to pool a greater amount of moisture. As a result, for example, even in a case where a procedure is to be performed for a long period of time in an environment where there is little moisture in the surroundings such as inside the lesion of a chronic total occlusion (CTO), by supplying a sufficient amount of moisture to the hydrophilic film, it becomes possible to continue to exhibit lubricity.

### Brief Description of Drawings

Fig. 1 is an explanatory diagram illustrating the configuration of a guide wire according to the first embodiment of the present invention.
Fig. 2 is an enlarged view of the coil body and the coating portion of the guide wire according to the first embodiment of the present invention.
Fig. 3 is an explanatory diagram illustrating the function of the spaces of the guide wire according to the first embodiment of the present invention. Enlarged views of the section outlined by the dotted line in Fig. 2 are illustrated in Fig. 3A, 3B. Furthermore, Fig. 3A illustrates a situation where moisture (blood) exists around the guide wire while Fig. 3B illustrates a situation in which the guide wire is placed in an environment where there is little moisture such as inside the lesion of a chronic total occlusion (CTO).
Fig. 4 is an enlarged view of the coil body and the coating portion of a guide wire according to the second embodiment of the present invention.
Fig. 5 is an enlarged view of the coil body and the coating portion of a guide wire according to the third embodiment of the present invention.

### Description of Embodiments

### A. First Embodiment:

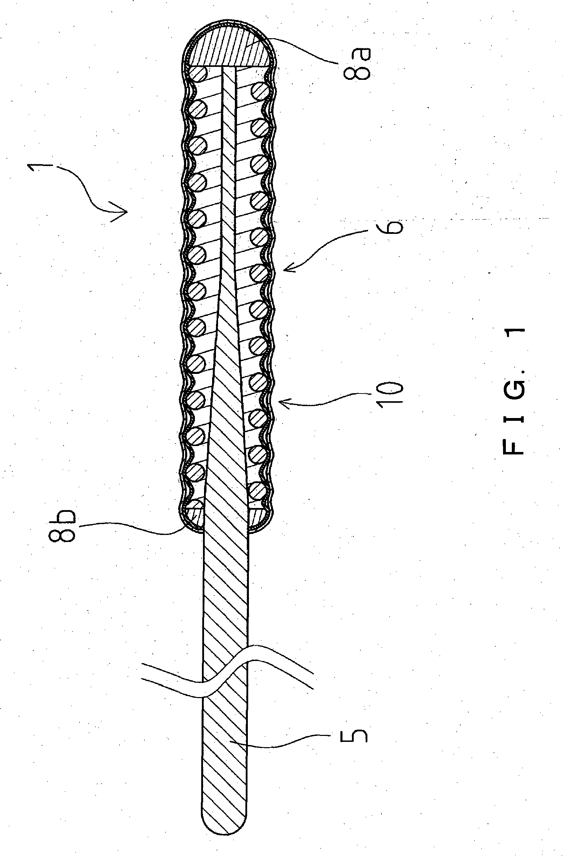

Various embodiments of a guide wire according to the present invention are explained below in order to clarify the details of the abovementioned present invention. Fig. 1 is an explanatory diagram illustrating the configuration of guide wire (1) according to the first embodiment of the present invention. Guide wire (1) is configured of such as core shaft (5) and coil body (6) which covers core shaft (5). Core shaft (5) and coil body (6) are joined to each other via a joint made of such as a brazing material. In this embodiment, the distal end portion of coil body (6) and the distal end portion of core shaft (5) are connected via joint (8a) while the base end portion of coil body (6) and the middle portion of core shaft (5) are connected via joint (8b).

Furthermore, with guide wire (1) of this embodiment, the surface of coil body (6) is covered with coating portion (10). Coating portion (10) is provided in order to ensure lubricity, when guide wire (1) is inserted into a blood vessel, by reducing the frictional resistance between the surface of guide wire (1) and the inner walls of the blood vessel.

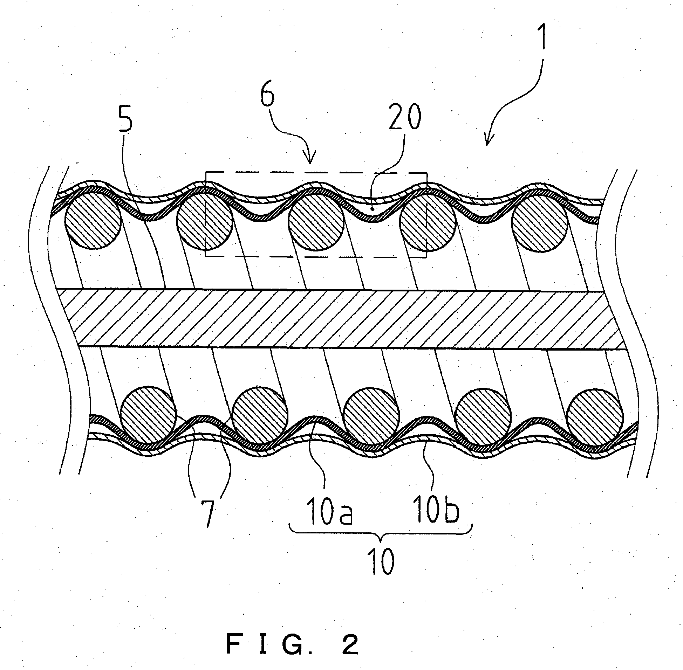

Fig. 2 is an enlarged view of coil body (6) and coating portion (10) of guide wire (1) according to the first embodiment of the present invention. As illustrated in the drawing, coil body (6) of guide wire (1) of this embodiment is formed in a so-called open-coiled shape where adjacent wires (7) are not in contact with each other. Furthermore, coating portion (10) on the surface of coil body (6) is formed by stacking a plurality of films (lower layer film (10a) and upper layer film (10b) in this embodiment).

In this configuration, with guide wire (1) of this embodiment, lower layer film (10a) is a hydrophobic film and upper layer film (10b) is a hydrophilic film. In Fig. 2, the hydrophobic film (lower layer film (10a)) is illustrated as a film with a dark hatching while the hydrophilic film (upper layer film (10b)) is illustrated as a film with a light hatching. Furthermore, lower layer film (10a) of coating portion (10) is arranged to come between the wires of coil body (6) while space (20) is formed between upper layer film (10b) and the sections where lower layer film (10a) is arranged to come between the wires of coil body (6).

The hydrophobic lower layer film (10a) of this embodiment may be formed using, for example, a polyurethane-based resin, a polystyrene-based resin, a polyester-based resin, a polyamide-based resin, an acrylate-based resin, a polyethylene-based resin, a fluorine-based resin, carbodiimide, or a cellulose-based polymer. In addition, lower layer film (10a) may be formed using, for example, a monomer of methyl methacrylate, vinyl acetate, vinyl laurate, or vinyl stearate or a copolymer thereof.

Furthermore, lower layer film (10a) may also be formed as a mixture of the abovementioned materials.

In addition, the hydrophilic upper layer film (10b) of this embodiment may be formed with, for example, polyvinyl alcohol, polyvinyl pyrrrolidone, polyethylene glycol, polyacrylamide, polyacrylic acid, sodium polyacrylate, poly-(2-hydroxyethyl methacrylate), a maleic acid copolymer, an ethylene vinyl alcohol copolymer, a monomer of 2-methacryloyloxyehtyl phosphorylcholine or a copolymer thereof, a (2-hydroxyethyl methacrylate)-styrene block copolymer, various synthetic polypeptides, collagen, hyaluronic acid, a cellulose-based polymer, or a mixture of any of these.

Furthermore, the hydrophobic lower layer film (10a) of this embodiment may be a composition that consists of a hydrophilic material (for example, polyvinyl alcohol) and a hydrophobic material (for example, a cross-linking agent such as carbodiimide) and exhibits a hydrophobic property as a whole due to the mixing ratio of the hydrophobic material being high.

In addition, the hydrophilic upper layer film (10b) of this embodiment may be a composition that consists of a hydrophilic material (for example, polyvinyl alcohol) and a hydrophobic material (for example, a cross-linking agent such as carbodiimide) and exhibits a hydrophilic property as a whole due to the mixing ratio of the hydrophilic material being high.

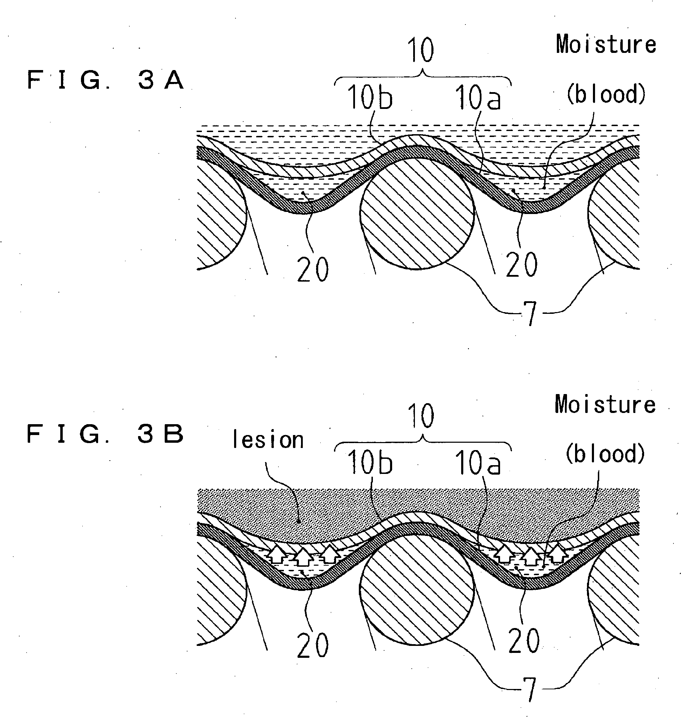

Fig. 3 is an explanatory diagram illustrating the function of space (20) of guide wire (1) according to the first embodiment of the present invention. Enlarged views of the section outlined by the dotted line in Fig. 2 are illustrated in Fig. 3A, 3B. Furthermore, Fig. 3A illustrates a situation where moisture (blood) exists around guide wire (1) while Fig. 3B illustrates a situation in which guide wire (1) is placed in an environment where there is little moisture such as inside the lesion of a chronic total occlusion (CTO).

As illustrated in Fig. 3A, in a case where moisture (blood) exists around guide wire (1), by moisture being constantly supplied to the hydrophilic upper layer film (10b), lubricity of upper layer film (10b) is exhibited. In addition, a part of this moisture (blood) permeates the hydrophilic upper layer film (10b) and stays pooled in space (20).

Meanwhile, as illustrated in Fig. 3B, in a case where guide wire (1) exists in an environment where there is little moisture such as inside the lesion of a chronic total occlusion (CTO), there is almost no supply of moisture to the hydrophilic upper layer film (10b) from the outside. However, as moisture is pooled in space (20) of coating portion (10), this moisture is supplied to the hydrophilic upper layer film (10b).

As a result, even in a case where guide wire (1) is placed in an environment where there is little moisture in its surroundings, it becomes possible to sufficiently exhibit the lubricity of the hydrophilic upper layer film (10b).

The above explains the function of space (20) in a case where guide wire (1) is inserted such as inside the lesion of a chronic total occlusion (CTO). Here, in cases other than that mentioned in the above, for example in a case where guide wire (1) is temporarily removed from the blood vessel during the procedure, due to the moisture around guide wire (1) no longer existing, the supply of moisture to the hydrophilic film (upper layer film (10b)) from the outside ceases to exist.

Even in such case, with guide wire (1) of this embodiment, as the moisture pooled in space (20) of coating portion (10) is supplied to the hydrophilic upper layer film (10b), it becomes possible to sufficiently exhibit the lubricity of the hydrophilic upper layer film (10b).

In addition, in a procedure where guide wire (1) and a catheter are used together, in a case where guide wire (1) and the catheter are inserted into a flexure of a blood vessel, coating portion (10) of guide wire (1) is pressed strongly against the inner walls of the catheter. As a result, it is possible that the moisture contained in the hydrophilic film (upper layer film (10b)) is discharged to the outside.

Even in such case, by the moisture pooled in space (20) of coating portion (10) being supplied to the hydrophilic upper layer film (10b), it becomes possible to sufficiently exhibit the lubricity of upper layer film (10b).

Along with the abovementioned first embodiment, there exist other related embodiments. The following are brief explanations of these other embodiments. It shall be noted that in the following explanations, configurations that are identical to guide wire (1) of the first embodiment are noted with the same numbers and detailed explanations thereof are omitted.

### B. Second Embodiment:

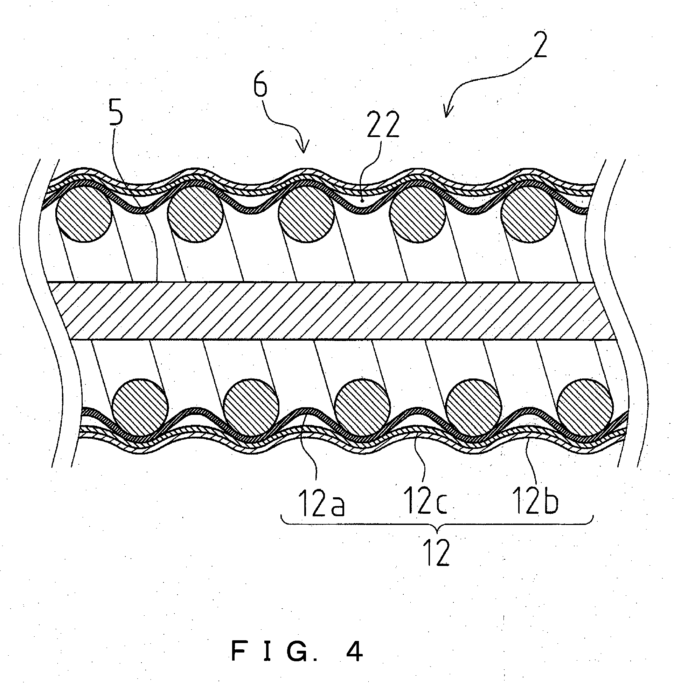

Fig. 4 is an enlarged view of coil body (6) and coating portion (12) of guide wire (2) according to the second embodiment. With guide wire (2) of this embodiment, coating portion (12) which covers coil body (6) is configured of 3 layers of films (lower layer film (12a), middle layer film (12c), upper layer film (12b)). Of these, lower layer film (12a) is formed of a hydrophobic film while middle layer film (12c) and upper layer film (12b) are formed of a hydrophilic film.

In addition, with this embodiment, upper layer film (12b) is formed of a material having a higher degree of water retentivity than middle layer film (12c). In Fig. 4, a further lighter hatching than middle layer film (12c) is used for upper layer film (12b) and by this, it is illustrated that the water retentivity of upper layer film (12b) is higher than that of middle layer film (12c).

Furthermore, in this embodiment, as one example, a hydrophilic polyvinyl alcohol is used for middle layer film (12c) while a hydrophilic hyaluronic acid having a high degree of water retentivity is used for upper layer film (12b).

Apart from the abovementioned point, guide wire (2) of this embodiment is similar to guide wire (1) according to the first embodiment. Namely, core shaft (5) is inserted through the bore of coil body (6). In addition, space (22) for pooling moisture is formed between middle layer film (12c) and the sections where lower layer film (12a) of coating portion (12) is arranged to come between wires (7) of coil body (6).

Also with such guide wire (2) of this embodiment, in an environment where there is little moisture in the surrounding thereof, by supplying the moisture pooled in space (22) to the hydrophilic upper layer film (12b), it is possible to sufficiently exhibit the lubricity thereof.

In addition, with guide wire (2) of this embodiment, since the hydrophilic film of coating portion (12) is multi-layered (having 2 layers in this embodiment) and upper layer film (12b) has a higher retentivity than middle layer film (12c), it is possible to ensure that the moisture pooled in space (22) stays pooled within space (22).

Here, for example in a case where guide wire (2) (and coil body (6)) is bent within a blood vessel, due to space (22) deforming, the moisture is inclined to be pushed outside from within space (22). Even in such case, with guide wire (2) of this embodiment, due to the high degree of water retentivity of upper layer film (12b), it is possible to ensure that the moisture of space (22) stays pooled within space (22). As a result, in a case when the amount of moisture surrounding guide wire (2) is reduced, it becomes possible to ensure that moisture is supplied to the hydrophilic upper layer film (12b).

### C. Third Embodiment:

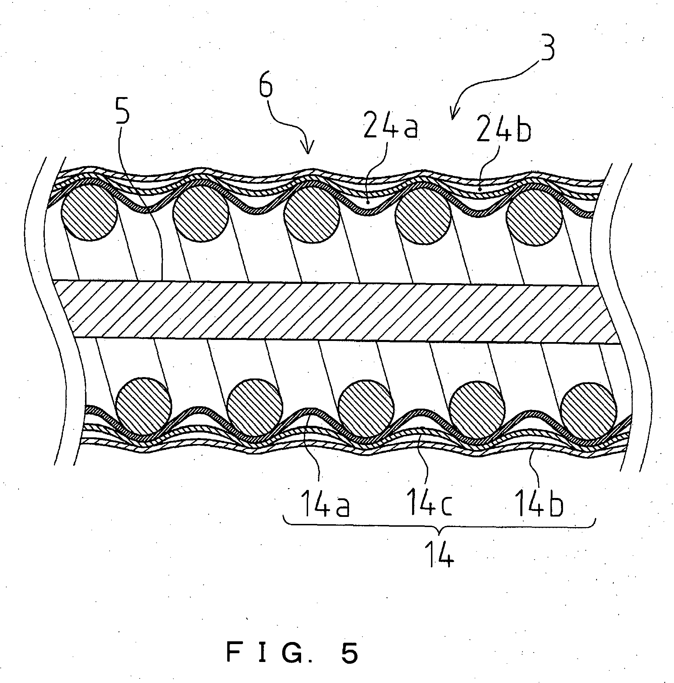

Fig. 5 is an enlarged view of coil body (6) and coating portion (14) of guide wire (3) according to the third embodiment. With guide wire (3) of this third embodiment, similar to guide wire (2) of the abovementioned second embodiment, coating portion (14) is of a structure having 3 layers consisting of a hydrophobic lower layer film (14a), a hydrophilic middle layer film (14c), and a hydrophilic upper layer film (14b). In addition, core shaft (5) is inserted through the bore of coil body (6).

Meanwhile, with guide wire (3) of the third embodiment, not only is space (24a) formed between lower layer film (14a) and middle layer film (14c) but space (24b) is formed also between middle layer film (14c) and upper layer film (14b).

With such guide wire (3) of this embodiment, similar to with the abovementioned guide wire (1) of the first embodiment and guide wire (2) of the second embodiment, in an environment where there is little moisture in the surrounding thereof, by supplying the moisture pooled in spaces (24a, 24b) to the hydrophilic upper layer film (14b), it is possible to sufficiently exhibit the lubricity thereof.

Especially with guide wire (3) of this embodiment, as not only space (24a) is formed between lower layer film (14a) and middle layer film (14c) but space (24b) is formed between middle layer film (14c) and upper layer film (14b), since there is a greater number of spaces, it is possible for a greater amount of moisture to be pooled in the spaces. As a result, for example, even in a case where a procedure is to be performed for a long period of time in an environment where there is little moisture such as inside the lesion of a chronic total occlusion (CTO), by continuing to supply moisture to the hydrophilic upper layer film (14b), it becomes possible to continue to exhibit lubricity.

In addition, with guide wire (3) of this embodiment, upper layer film (14b) is lifted up by the 2 spaces (spaces (24a, 24b)) and due to this the surface of upper layer film (14b) is a surface having a very small amount of unevenness. Accordingly, as it is possible to suppress the catching of upper layer film (14b) onto the outside, it becomes possible to improve lubricity within a blood vessel.

Although guide wire of various embodiments were explained in the above, the present invention is not limited to the abovementioned embodiments and it is possible for the present invention to be implemented in various manners without departing from the gist thereof. For example, with the abovementioned guide wires of the various embodiments, the hydrophilic film which configures the coating portion was explained as being that having 1 layer or 2 layers (refer to Fig. 2 through Fig. 5).

However, this hydrophilic film may be that having 3 or more layers (drawing omitted).

Understandably, if the number of hydrophilic films is too many, there is the possibility that such will inhibit the intake of moisture to within the space. Therefore, as in the abovementioned various embodiments, it is preferable that the hydrophilic film which configures the coating portion is of 1 layer or approximately 2 layers.

In addition, with the abovementioned guide wires of the various embodiments, the coil body was explained as being formed in a so-called open-coiled shape where adjacent wires are not in contact with each other. However, the coil body may be formed in a so-called close-coiled shape where adjacent wires are in contact with each other (drawing omitted).

However, since an open-coiled coil body allows for larger spaces to be formed, as a result, it becomes possible to sufficiently pool moisture to supply to the hydrophilic film of the coating portion. Therefore, as in the abovementioned guide wires of the various embodiments, it is preferable that the coil body is formed to be open-coiled.

### Reference Signs List

- 1, 2, 3: Guide wire
- 5: Core shaft
- 6: Coil body
- 7: Wire
- 8a, 8b: Joint
- 10, 12, 14: Coating portion
- 10a, 12a, 14a: Lower layer film
- 12c, 14c: Middle layer film
- 10b, 12b, 14b: Upper layer film
- 20, 22, 24a, 24b: Space

## Claims

1. A guide wire (1; 2; 3) comprising a core shaft (5), a coil body (6) which covers said core shaft (5), and a coating portion (10; 12; 14) which covers said coil body (6), wherein:
said coating portion (10; 12; 14) is formed by stacking two films (10a, 10b; 12a, 12b; 12c; 14a, 14b, 14c);
the lowermost layer film (10a; 12a; 14a) of said coating portion (10; 12; 14) is a hydrophobic film;
the upper layer film (10b; 12c, 12b; 14c, 14b) above the lowermost layer film (10a; 12a; 14a) of said coating portion (10b 12; 14) is a hydrophilic film;
said hydrophobic film (10b; 12a; 14a) is arranged to come between wires (7) of said coil body (6); **characterized in that**
a space (20; 22; 24a) is formed between the hydrophilic film (10b; 12c, 12b; 14c, 14b) and the sections of the hydrophobic film (10a; 12a; 14a) where said hydrophobic film (10a; 12a; 14a) is arranged to come between the wires (7) of said coil body (6).

2. A guide wire (2; 3) according to Claim 1, wherein:
the hydrophilic film (12c, 12b; 14c, 14b) of said coating portion (12; 14) has 2 or more layers (12c, 12b; 14c, 14b) wherein the hydrophilic film (12b; 14b) of the top layer has a higher degree of water retentivity than the hydrophilic film (12c, 14c) of the bottom layer.

3. A guide wire (3) according to Claim 2, wherein:
in sections where the film of said coating portion (14) is arranged to come between the wires (7) of said coil body (6), spaces (24b) are also formed between said hydrophilic films (14c, 14b).

## Patentansprüche

1. Führungsdraht (1; 2; 3) mit einem Kern (5), einer den Kern (5) umgebenden Spule (6) und einer die Spule (6) umhüllenden Beschichtung (10; 12; 14), wobei:
die Beschichtung (10; 12; 14) aus zwei übereinander liegenden Filmen (10a, 10b; 12a, 12b; 12c; 14a, 14b, 14c) gebildet ist;
der unterste Film (10a; 12a; 14a) der Beschichtung (10; 12; 14) ein hydrophober Film ist;
der über dem untersten Film (10a; 12a; 14a) liegende obere Film (10b; 12c, 12b; 14c, 14b) der Beschichtung (10b; 12; 14) ein hydrophiler Film ist;
der hydrophobe Film (10b; 12a; 14a) so angeordnet ist, dass er zwischen die Drähte (7) der Spule (6) tritt, **dadurch gekennzeichnet, dass**
zwischen dem hydrophilen Film (10b; 12c, 12b; 14c, 14b) und den Bereichen des hydrophoben Films (10a; 12a; 14a), in denen der hydrophobe Film (10a; 12a; 14a) so angeordnet ist, dass er sich die Drähte (7) der Spule (6) tritt, ein Raum (20; 22; 24a) geschaffen ist.

2. Führungsdraht (2; 3) nach Anspruch 1, wobei:
der hydrophile Film (12c, 12b; 14c, 14b) der Beschichtung (12; 14) zwei oder mehrere Lagen (12c, 12b; 14c, 14b) aufweist, wobei der hydrophile Film (12b; 14b) der oberen Lage ein höheres Wasserrückhaltevermögen hat als der hydrophile Film (12c, 14c) der unteren Lage.

3. Führungsdraht (3) nach Anspruch 2, wobei:
in den Bereichen, in denen der Film der Beschichtung (14) so angeordnet ist, dass er zwischen die Drähte (7) der Spule (6) tritt, auch zwischen den hydrophilen Filmen (14c, 14b) Räume (24b) geschaffen sind.

## Revendications

1. Fil-guide (1 ; 2 ; 3) comprenant une tige centrale (5), un corps de bobine (6) qui couvre ladite tige centrale (5), et une partie de revêtement (10 ; 12 ; 14) qui couvre ledit corps de bobine (6), dans lequel :
ladite partie de revêtement (10 ; 12 ; 14) est formée par empilement de deux films (10a, 10b ; 12a, 12b ; 12c ; 14a, 14b, 14c) ;
le film de la couche inférieure (10a ; 12a ; 14a) de ladite partie de revêtement (10 ; 12 ; 14) est un film hydrophobe ;
le film de la couche supérieure (10b ; 12c, 12b ; 14c, 14b) au-dessus du film de la couche inférieure (10a ; 12a ; 14a) de ladite partie de revêtement (10b ; 12 ; 14) est un film hydrophile ;
ledit film hydrophobe (10b ; 12a ; 14a) est agencé pour venir entre des fils (7) dudit corps de bobine (6) ; **caractérisé en ce que**
un espace (20 ; 22 ; 24a) est formé entre le film hydrophile (10b ; 12c, 12b ; 14c, 14b) et les sections du film hydrophobe (10a ; 12a ; 14a) où ledit film hydrophobe (10a ; 12a ; 14a) est agencé pour venir entre les fils (7) dudit corps de bobine (6).

2. Fil-guide (2 ; 3) selon la revendication 1, dans lequel :
le film hydrophile (12c, 12b ; 14c, 14b) de ladite partie de revêtement (12 ; 14) a 2 couches (12c, 12b ; 14c, 14b) ou plus dans lequel le film hydrophile (12b ; 14b) de la couche supérieure a un degré de rétention d'eau supérieur à celui du film hydrophile (12c, 14c) de la couche inférieure.

3. Fil-guide (3) selon la revendication 2, dans lequel :
dans les parties où le film de ladite partie de revêtement (14) est agencé pour venir entre les fils (7) dudit corps de bobine (6), des espaces (24b) sont également formés entre lesdits films hydrophiles (14c, 14b).
